# EUROPEAN PATENT SPECIFICATION

(11) **EP 1 600 446 B1**
(45) Date of publication and mention of the grant of the patent: **17.10.2007**
(21) Application number: 04291268.3
(22) Date of filing: 18.05.2004
(51) Int. Cl.: C07D 313/08

(54) **New 3,3-dimethyl-5-cyano-benzoxepine derivatives useful for the preparation of 5-formyl-benzoxepine derivatives**
Neue 3,3-Dimethyl-5-Cyano-Benzoxepinderivate verwendbar zur Herstellung von 5-Formyl-Benzoxepinderivaten
Nouveaux derives de 3,3-dimethyl-5-cyano-benzoxepine utilisable pour la préparation des derives de 5-formyl-benzoxepine

(43) Date of publication of application: 30.11.2005
(73) Proprietor: Merck Sante, 69008 Lyon (FR)
(72) Inventor: Brunet, Michel, 69780 Toussieu (FR); Le Borgne, Guy, 45300 Pithiviers le Vieil (FR)
(74) Representative: Bernasconi, Jean Raymond

(56) References cited:
- EP-A- 1 140 893
- PRYDE D C ET AL: "Synthesis of 2-Tetralones via a Novel 1,2-Carbonyl Transposition of 1-Tetralones" TETRAHEDRON LETTERS, ELSEVIER SCIENCE PUBLISHERS, AMSTERDAM, NL, vol. 37, no. 19, 6 May 1996 (1996-05-06), pages 3243-3246, XP004029369 ISSN: 0040-4039
- ZIEGLER F E ET AL: "A FORMAL STEREO CONTROLLED SYNTHESIS OF RACEMIC ESTRONE EMPLOYING THE TRI METHYLSILYL CYANOHYDRIN COPE REARRANGEMENT" TETRAHEDRON LETTERS, vol. 22, no. 13, 1981, pages 1179-1182, XP002294537 ISSN: 0040-4039

## Description

### FIELD OF THE INVENTION

The present invention relates to 3,3-dimethyl-5-cyano-benzoxepine derivatives and their use for the preparation of 3,3-dimethyl-5-formyl-2,3-dihydrobenzoxepine derivatives.

### BACKGROUND OF THE INVENTION

3,3-dimethyl-5-formyl-2,3-dihydrobenzoxepine derivatives (formula II): 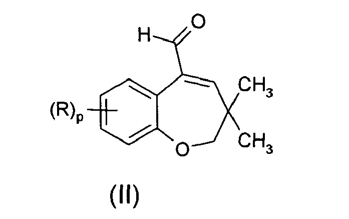 are disclosed in EP 1140893 B1 and US 6596758 patents as intermediates for the preparation of 5-(3,3-dimethyl-2,3-dihydro benzoxepin-5-yl)-2,4-pentadienoic acid derivatives, which in turn are useful for treating dyslipidemias, atherosclerosis and diabetes.

In these patents, compounds of formula II are prepared according to the following scheme :

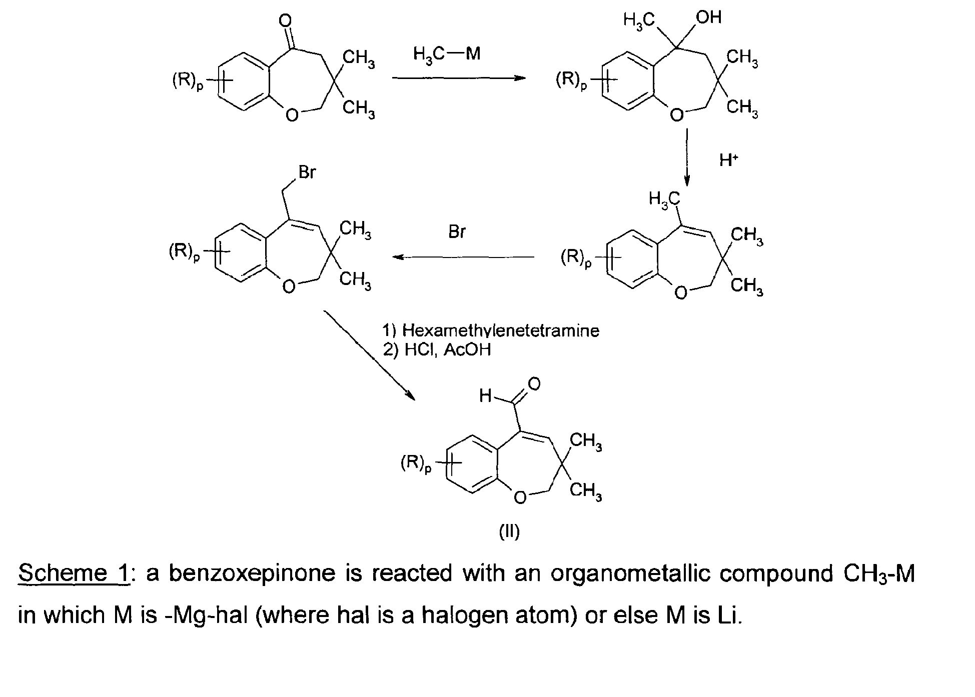

This synthetic method involves four chemical steps starting from benzoxepinone and the yields, as reported, are moderate.

Furthermore, this synthetic pathway cannot be easily scaled up to commercial implementation.

It now has been found a novel improved synthetic route for preparing the compounds of formula (II) which is unexpectedly applicable at industrial scale.

Advantageously, the compounds of formula (II) can be obtained in only three steps, each being characterized by high yields.

As another advantage, the invention provides an economical and efficient route for preparing the compounds of formula (II).

According to the present invention, compounds of formula (II) are prepared from new compounds of formula (I).

Thus, in one aspect, the present invention is related to compounds of general formula (I) :

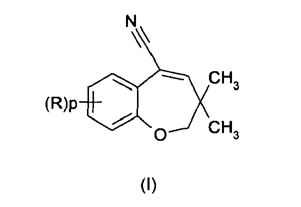

Each of R is independently chosen from a halogen atom; a cyano group; a nitro group; a carboxy group; an optionally halogenated (C₁-C₁₈)alkoxycarbonyl group; an Rₐ-CO-NH- or RₐR_{b}N-CO- group [in which Rₐ and R_{b} independently represent optionally halogenated (C₁-C₁₈)alkyl; a hydrogen atom; (C₆-C₁₀)aryl or (C₆-C₁₀)aryl(C₁-C₅)alkyl (where the aryl parts are optionally substituted by a halogen atom, by an optionally halogenated (C₁-C₅)alkyl group or by an optionally halogenated (C₁-C₅)alkoxy group); (C₃-C₁₂)cycloalkyl optionally substituted by a halogen atom, by an optionally halogenated (C₁-C₅)alkyl group or by an optionally halogenated (C₁-C₅)alkoxy group]; an optionally halogenated (C₁-C₁₈)alkyl group; optionally halogenated (C₁-C₁₈)alkoxy; and (C₆-C₁₀)aryl, (C₆-C₁₀)aryl(C₁-C₅)alkyl, (C₆-C₁₀)aryloxy, (C₃-C₁₂)cyclo-alkyl, (C₃-C₁₂)cycloalkenyl, (C₃-C₁₂)cycloalkyloxy, (C₃-C₁₂)cycloalkenyloxy ; (C₆-C₁₀)aryloxycarbonyl or (C₆-C₁₀)arylcarbonyl; in which the aryl, cycloalkyl and cycloalkenyl parts are optionally substituted by a halogen atom, by an optionally halogenated (C₁-C₅)alkyl or by an optionally halogenated (C₁-C₅)alkoxy; p represents 0, 1, 2, 3 or 4;

The formula (I) encompasses all types of geometric isomers and stereoisomers of the compounds of formula (I) or mixtures thereof.

As used above and throughout the description of the invention, the following terms, unless otherwise indicated, shall be understood to have the following meanings.

"Alkyl" means an aliphatic hydrocarbon group which may be straight or branched, having 1 to 18 carbon atoms in the chain. Preferred alkyl groups have 1 to 12 carbon atoms in the chain.

"Branched alkyl" means that one or more lower alkyl groups such as methyl, ethyl or propyl are attached to a linear alkyl chain.

"Lower alkyl" means an alkyl group with 1 to about 4 carbon atoms in the chain which may be straight or branched.

Exemplary alkyl groups include methyl, ethyl, propyl, isopropyl, butyl, tert-butyl, isobutyl, pentyl, hexyl, heptyl, octyl, nonyl, decyl, undecyl, dodecyl, tridecyl, tetradecyl, pentadecyl, hexadecyl, heptadecyl or octadecyl.

The alkyl group may be substituted by one or more halogen atoms representing thus an "halogenoalkyl" group.

"Halogen atoms" means fluorine, chlorine, bromine or iodine atoms. Preferred are fluorine, chlorine or bromine atoms and more preferred is fluorine atoms.

The "halogenoalkyl" groups may thus refer to "perfluoroalkyl", which means groups corresponding to the formula "-CₙF₂ₙ₊₁" wherein n represents 1 to 18.

Examples of perfluoroalkyl groups are pentafluoroethyl or trifluoro-methyl.

"Alkoxy" means an alkyl-O- group wherein the alkyl group is as herein described. Exemplary alkoxy groups include methoxy, ethoxy, isopropyloxy, butoxy and hexyloxy radicals.

"Cycloalkyl" means a non-aromatic mono- or multicyclic ring system of about 3 to 12 carbon atoms. Preferred ring sizes of the ring system include about 3 to 8 and more preferably 5 to 6 ring atoms. The cycloalkyl is optionally substituted with one or more "ring system substituents" which may be the same or different, and are as defined herein. Exemplary monocyclic cycloalkyl include cyclopropyl, cyclobutyl, cyclopentyl, cyclohexyl, cycloheptyl, cyclooctyl, cyclononyl, cyclodecyl, cycloundecyl, cyclododecyl and the like.

Exemplary multicyclic cycloalkyl include 1-decalyn, norbornyl and the like.

"Cycloalkenyl" means a non-aromatic mono- or multicyclic ring system of about 3 to about 12 carbon atoms, preferably of about 5 to about 10 carbon atoms, and which contain at least one carbon-carbon double bond. Preferred ring size of rings of the ring system include about 5 to about 6 ring atoms. The cycloalkenyl is optionally substituted with one or more "ring system substituents" which may be the same or different, and are as defined herein. Exemplary monocyclic cycloalkenyl include cyclopentenyl, cyclo-hexenyl, cycloheptenyl and the like. An exemplary multicyclic cycloalkenyl is norbornylenyl.

"Aryl" means an aromatic monocyclic or multicyclic ring system of about 6 to about 10 carbon atoms. The aryl is optionally substituted with one or more "ring system substituents" which may be the same or different and are as defined herein. Exemplary aryl groups include phenyl or naphtyl, or substituted phenyl or substituted naphtyl.

"Alkenyl" means an aliphatic hydrocarbon group containing one or more carbon-carbon double bond and which may be straight or branched, having about 2 to about 12 carbon atoms in the chain, and more preferably about 2 to about 4 carbon atoms in the chain.

"Branched alkenyl" means that one or more lower alkyl or alkenyl groups such as methyl, ethyl or propyl are attached to a linear alkenyl chain. "Lower alkenyl" means about 2 to about 4 carbon atoms in the chain, which may be straight or branched. The alkenyl group may be substituted by one or more halogen atoms. Exemplary alkenyl groups include ethenyl, propenyl, n-butenyl, i-butenyl, 3-methylbut-2-enyl, n-pentenyl, heptenyl, octenyl, cyclohexyl-butenyl and decenyl.

"Aryloxy" means an aryl-O- group wherein the aryl group is as defined herein. Exemplary groups include phenoxy and 2-naphtyloxy.

"Aryloxycarbonyl" means an aryl-O-CO- group wherein the aryl group is as defined herein. Exemplary aryloxycarbonyl groups include phenoxy-carbonyl and naphtoxycarbonyl.

"Arylcarbonyl" refers to an aryl-CO- group wherein the aryl group is as defined herein.

Exemplary arylcarbonyl group includes benzoyl.

The (C₆-C₁₀) aryl, (C₃-C₁₂) cycloalkyl, (C₃-C₁₂) cycloalkenyl are optionally substituted by one or more "ring system substituents".

"Ring system substituents" mean substituents attached to aromatic or non-aromatic ring systems, inclusive of halogen atoms, an optionally halogenated (C₁-C₅) alkyl, or an optionally halogenated (C₁-C₅) alkoxy, halogen, alkyl and alkoxy being as defined herein,

The wording "in which the aryl, cycloalkyl and cycloalkenyl parts are optionally substituted by a halogen atom, by an optionally halogenated (C₁-C₅)alkyl or by an optionally halogenated (C₁-C₅)alkoxy" means that the aryl, cycloalkyl, cycloalkenyl groups are optionally substituted by one or more substituents selected from the group consisting of :
- halogen atoms ;
- alkyl groups optionally substituted by one or more halogen atoms, and
- alkoxy groups optionally substituted by one or more halogen atoms.

The wording "optionally halogenated" means, in the context of the description, optionally substituted by one or more halogen atoms.

Preferably, each of R independently represents a halogen atom, an optionally substituted halogenated (C₆-C₁₀) arylcarbonyl, an optionally halogenated (C₁-C₁₈) alkyl, an optionally halogenated (C₁-C₁₈) alkoxy, or an optionally halogenated (C₆-C₁₀) aryl.

Examples of preferred halogen atoms include fluorine, bromine and chlorine atoms.

Examples of optionally substituted halogenated (C₆-C₁₀) arylcarbonyl include the groups ortho, meta or para chlorobenzoyl, or ortho, meta, para-bromobenzoyl.

Examples of preferred optionally halogenated (C₁-C₁₈) alkyl include notably perfluoroalkyl groups such as trifluoromethyl.

Examples of preferred optionally halogenated (C₁-C₁₈) alkoxy include notably optionally halogenated (C₁-C₆) alkoxy, particularly (C₁-C₄) alkoxy such as methoxy, ethoxy, isopropyloxy, n-butoxy, isobutoxy.

Examples of particularly preferred optionally halogenated (C₆-C₁₀) aryl include notably phenyl.

More preferably, R represents a (C₁-C₁₈) alkoxy group, more preferably a (C₁-C₄) alkoxy group and, most preferably, a methoxy group.

Preferably, p is 1 or 2 and more preferably 1.

R may be located in position 6, 7, 8 or 9 on the benzoxepine structure, as represented hereafter.

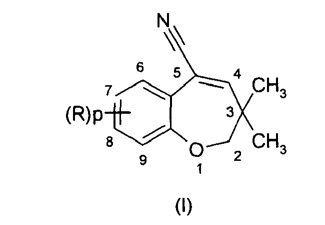

Preferred compounds of formula (I) are chosen from:
3,3-dimethyl-5-cyano-7-bromo-2,3-dihydrobenzoxepine,
3,3-dimethyl-5-cyano-9-methoxy-2,3-dihydrobenzoxepine,
3,3-dimethyl-5-cyano-7,8-dichloro-2,3-dihydrobenzoxepine,
3,3-dimethyl-5-cyano-7-fluoro-8-chloro-2,3-di-hydrobenzoxepine,
3,3-dimethyl-5-cyano-7-(para-chlorobenzoyl)-2,3-dihydrobenzoxepine,
3,3-dimethyl-5-cyano-7-trifluoromethyl-2,3-di-hydrobenzoxepine,
3,3-dimethyl-5-cyano-7-fluoro-2,3-dihydrobenzoxepine,
3,3-dimethyl-5-cyano-7-chloro-2,3-dihydrobenzoxepine,
3,3-dimethyl-5-cyano-7,8-dimethoxy-2,3-dihydro-benzoxepine,
3,3-dimethyl-5-cyano-7-phenyl-2,3-dihydrobenzoxepine,
3,3-dimethyl-5-cyano-2,3-dihydrobenzoxepine,
3,3-dimethyl-5-cyano-7-methoxy-2,3-dihydrobenzoxepine
as well as their geometric isomers and stereoisomers or mixtures thereof.

A more preferred compound of formula (I) is 3,3-dimethyl-5-cyano-7-methoxy-2,3-dihydrobenzoxepine : 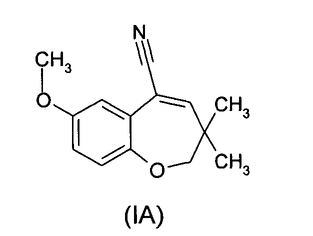 as well as its geometric isomers and stereoisomers or mixtures thereof.

### Methods for preparing compounds of formula (II) starting from compounds of formula (I)

According to the invention, the compounds of formula (I) are used for the preparation of compounds of formula (II) according to scheme 2 :

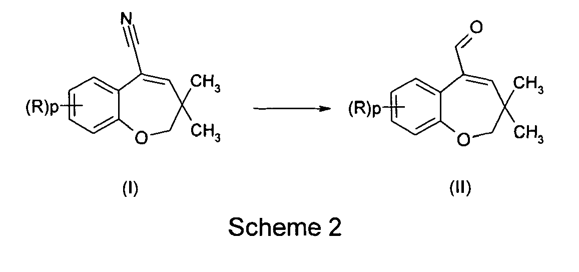

Thus, in another aspect, the present invention is directed to a method for preparing compounds of formula (II), comprising :
a) reacting the compounds of formula (I) with a reducing agent ; and optionally
b) hydrolyzing the resulting mixture ; and optionally
c) isolating the obtained compound of formula (II).

### Step a)

The conversion of the compound of formula (I) into the compound of formula (II) is carried out in the presence of a reducing agent. There is no particular restriction on the nature of the reducing agent used in this reaction and any reducing agent conventionally used in a reaction of this type may equally be used here, provided that it has no adverse effect on other parts of the molecule.

Suitable reducing agents for reducing the nitrile compound of formula (I) to aldehyde include metal hydride reducing agents such as LiAlH₄, NaAlH₄, LiAlH (Oalkyl)₃, LiAlH₂(Oalkyl)₂, LiAlH(NR₂)₃ where R is H or an alkyl group and iPr₂AlH, iBu₂AlH, also called DIBAL-H, the DIBAL-H being particularly preferred.

Another suitable method for reducing the nitrile to aldehyde, known as the Stephen reduction, involves reacting the nitrile with HCl and SnCl₂. More particularly, it generally involves treating the nitrile with HCl, reducing the formed intermediate with SnCl₂ and hydrolyzing the obtained imine to the corresponding aldehyde.

The conditions described in the following publications may be applied or adapted to the reduction of (I) to (II) when implementing the indicated reducing agents :
- iPr₂AlH (Angew. Chem. Int. Ed., 1973, 12, 497);
- iBu₂AlH (many references, notably J. Org. Chem., 1970, 35, 858, Marshall, Andersen, Schlicher) ;
- LiAH(OEt)₃ (J. Amer. Chem. Soc., 1964, 86, 1085, Brown et Garg) ;
- LiAlH₂(OEt)₂ (J. Org. Chem., 1979, 44, 4603) ;
- NaAlH₄ (Bull. Acad. Sci. USSR, Div. Chem. Sci., 1964, 1415, Zakharkin, Maslin, Gavrilenko).

Other reactants may be used for the conversion of (I) into (II), such as Et₃SiH (J. Org. Chem., 1981, 46, 802), Ni Raney (Org. Synth., 1971, 51, 20, Van Es, Staskun) or Zinc-cobalamine (Helv. Chim. Acta, 1978, 61, 2560, Fischli).

The amount of reducing agent is for example 1.0 to 2 moles and more preferably 1.1 to 1.5 moles relative to 1 mole of compound (I).

There is no particular restriction on the nature of the solvent employed, provided that it has no adverse effect on the reactions or on the reagents involved.

Suitable solvents for step a) are aromatic solvents, ethers, halogenated hydrocarbons and aliphatic hydrocarbons and mixtures thereof.

Examples of aromatic solvents are benzene, toluene, xylene and ethylbenzene.

Examples of ethers include dialkyl ethers such as diethyl ether, dibutyl ether, dioxane, tetrahydrofurane.

Examples of halogenated hydrocarbons include notably dichloromethane, chloroforme, 1,2-dichloroethane.

Examples of aliphatic hydrocarbons include notably pentane, hexane, heptane and octane.

Preferably, anhydrous conditions are used.

The reaction of step a) can take place over a wide range of temperatures, and the precise reaction temperature is not critical to the invention. In general, it has been found convenient to carry out the reaction at a temperature from about -20°C to room temperature and preferably from -10°C to 0°C.

The time required for the reaction may also vary widely, depending on many factors, notably the reaction temperature and the nature of the reagents. However, provided that the reaction is effected under the preferred conditions outlined above, a period from about 5minutes to about 20 hours will usually be sufficient.

### Step b)

In accordance with a preferred embodiment, the method for preparing the compound of formula (II) further comprises the step of hydrolyzing the compound obtained in step a).

Indeed, when reducing nitrils to aldehydes involve using a metal hydride, the method may generally lead to the intermediate formation of an imine derivative that may be hydrolyzed in order to give the aldehyde (II). This hydrolysis is preferably performed *in situ*. The following scheme 3 is given as an illustration of this reaction pathway and is not to be considered as limiting the invention in its scope.

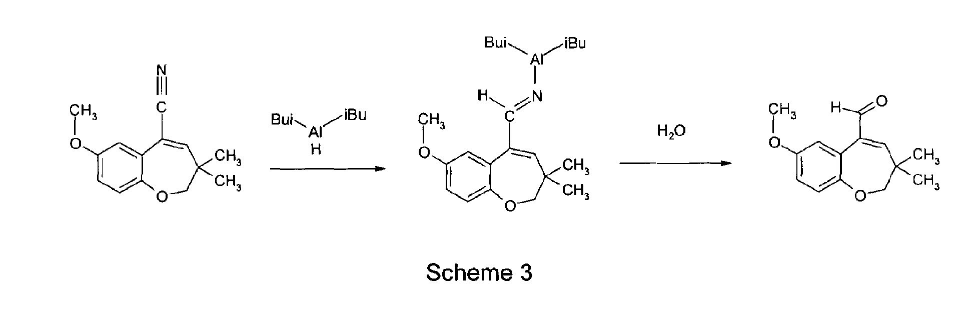

Preferably, the hydrolysis is performed under acid conditions.

Suitable acids for the hydrolysis of the compounds obtained in step a) include inorganic acids, such as hydrochloric acid, sulphuric acid, nitric acid and phosphoric acid ; sulfonic acids such as methanesulfonic acid, ethanesulfonic acid, benzenesulfonic acid and paratoluenesulfonic acid.

Inorganic acids are most preferred and notably hydrochloric acid.

Excess amount of acid is generally used and the amount of acid is for example 5 to 10 moles relative to 1 mole of compound (I).

In that context, the mixture is stirred, for example for 0.5 hour to 2 hours and preferably for 1 hour to 1.5 hour.

In that context, it has been found convenient to carry out the reaction at a temperature that does not exceed 40°C, for example from about room temperature to about 40°C.

The time required for each reaction may also vary widely, depending on many factors, notably the reaction temperature and the nature of reagents. However, provided that the reaction is effected under the preferred conditions outlined above, a period from about 0.5 hour to about 2 hours will usually be sufficient for the hydrolysis step.

### Step c)

The compounds thus prepared may be recovered from the reaction mixture by conventional means, for example the compounds may be recovered by distilling of the solvent from the reaction mixture or, if necessary, optionally after distilling of the solvent from the reaction mixture, pouring the residue into water, followed by extraction with a water-immiscible organic solvent and distilling of the solvent from the extract. Additionally, the product can, if desired, be further purified by various well known techniques, such as recrystallization, reprecipitation or the various chromatography techniques, notably column chromatography or preparative thin layer chromatography.

Preferred compounds of formula (II) which may conveniently be prepared starting from corresponding compounds of formula (I) according to the present invention can be chosen from the group consisting in:
3,3-dimethyl-5-formyl-7-bromo-2,3-dihydrobenzoxepine,
3,3-dimethyl-5-formyl-9-methoxy-2,3-dihydrobenzoxepine,
3,3-dimethyl-5-formyl-7,8-dichloro-2,3-dihydrobenzoxepine,
3,3-dimethyl-5-formyl-7-fluoro-8-chloro-2,3-di-hydrobenzoxepine,
3,3-dimethyl-5-formyl-7-(para-chlorobenzoyl)-2,3-dihydrobenzoxepine,
3,3-dimethyl-5-formyl-7-trifluoromethyl-2,3-di-hydrobenzoxepine,
3,3-dimethyl-5-formyl-7-fluoro-2,3-dihydrobenzoxepine,
3,3-dimethyl-5-formyl-7-chloro-2,3-dihydrobenzoxepine,
3,3-dimethyl-5-formyl-7,8-dimethoxy-2,3-dihydro-benzoxepine,
3,3-dimethyl-5-formyl-7-phenyl-2,3-dihydrobenzoxepine,
3,3-dimethyl-5-formyl-2,3-dihydrobenzoxepine,
3,3-dimethyl-5-formyl-7-methoxy-2,3-dihydrobenzoxepine.

### Methods for preparing the compounds of formula (I)

The compounds useful according to the invention may be prepared by the application or adaptation of known methods, by which are meant methods used heretofore or described in the literature, for example those described by R. C. Larock in *Comprehensive Organic Transformations,* VCH Publishers, 1989.

Thus, in a further aspect, the invention provides a method for preparing the compounds of formula (I) comprising :
i) reacting a compound of formula (III): 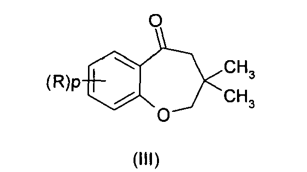 with CN- or a species providing CN⁻;
ii) hydrolyzing the resulting mixture ; and optionally
iii) isolating the obtained compound of formula (I).

### Step i)

The reaction of step i) consists in converting the cetone of formula (III) into the corresponding cyanohydrin. This reaction requires the presence of CN- ions; these may be provided in a free form in the reaction mixture or, alternatively, may be obtained by using a species providing such CN- ions. Such species include a alkali metal cyanide, such as NaCN or KCN, diethylaluminum cyanide (Et₂AlCN) or trialkyl- or triaryl-silylcyanide. Other suitable species include equivalents of trialkylsilylcyanide such as notably KCN-Me₃SiCl (Tetrahedron Asymmetry, 2001, 12(2), 279-286, Effenberger F., Oswald S.), LiCN-Me₃SiCl (Synthesis, 1986, 12, 1054-1055, Yoneda R., Santo K., Harusawa S., Kirihara T.).

Preferably, the reaction is carried out in the presence of trialkylsilylcyanide such as trimethylsilylcyanide (Me₃SiCN), trimethylsilylcyanide being most preferred.

When trialkylsilylcyanide or triarylsilylcyanide are used, it is particularly preferred to carry out the reaction of step i) in the presence of a Lewis acid or a base.

Examples of suitable basis include alkali metal hydrides, such as lithium hydride, sodium hydride and potassium hydride; (C₁-C₁₀) alkyllithium compounds such as methyllithium, butyllithium, hexyllithium ; alkali metal alkoxides such as sodium methoxide and sodium ethoxide ; and alkali metal carbonates such as potassium carbonate and sodium carbonate. Of these, the alkyllithium compounds and notably the butyllithium are particularly preferred.

Alternatively, other Lewis acids or bases may be used such as LiClO₄, LiBF₄, Zn(CN)₂, ZnI₂, KCN, Bu₄NCN, DABCO (diazabicyclooctane),Ti(OiPr)₄, CaF₂, Lewis acid-amberlite-trimethylsilyl triflate.

The amount of base is generally catalytic and is for example 0.01 to 0.5 moles and preferably 0.1 to 0.25 moles relative to 1 mole of compound (III).

There is no particular restriction on the nature of the solvent to be used, provided that it has no adverse effect on the reaction or on the reagent involved.

Examples of suitable solvents include hydrocarbons, such as hexane, cyclohexane, benzene, toluene and xylene; aprotic polar solvents such as N,N-dimethylformamide, N-methylpyrrolidone, dimethylsulfoxide, pyridine. Of these, hexane and pyridine are particularly preferred.

The reaction can take place over a wide range of temperatures, and the precise reaction temperature is not critical to the invention. In general, it has been found convenient to carry out the reaction at a temperature from about room temperature (20°C) to 150°C, and more preferably of from 20°C to 50°C.

The molar ratio of CN- or the species providing CN- relative to compound (III) may vary from 1.0 to 1.5 equivalent, preferably from 1.05 to 1.25.

### Step ii)

The reaction of the compound of formula (III) with CN- or CN- providing species generally lead to an intermediate cyanohydrin compound, which in turn leads after hydrolysis to the compound of formula (I).

This hydrolysis can be carried out *in situ,* straight after step i).

Examples of acids suitable for said hydrolysis include, but are not limited to, hydrochloric acid, sulphuric acid, nitric acid and phosphoric acid ; trifluoroacetic acid ; sulphonic acid, such as methane sulfonic acid, ethane sulfonic acid, benzene sulfonic acid and paratoluene sulfonic acid. In case trialkyl- or triarylsilylcyanide is used, the hydrolysis is preferably performed in the presence of a chlorinating agent such as phosphorus oxychloride (POCl₃), thionyl chloride (SOCl₂), sulfuryl chloride (SO₂Cl₂) ; or in the presence of trifluoroacetic acid (CF₃CO₂H), paratoluene sulphonic acid and hydrochloric acid (gaz).

The molar ration of said chlorinating agent relative to compound of formula (III) is from 1 to 2 equivalents, preferably 1.5 equivalents.

There is no particular restriction on the nature of the solvent to be used, provided that it has no adverse effect on the reaction or on the reagent involved. As step ii) can be conducted *in situ,* the same solvents as for step i) can be used.

Examples of suitable solvents include hydrocarbons, such as hexane, cyclohexane, benzene, toluene and xylene; aprotic polar solvents such as N,N-dimethylformamide, N-methylpyrrolidone, dimethylsulfoxide, pyridine. Of these, hexane and pyridine are particularly preferred.

The reaction can take place over a wide range of temperatures, and the precise reaction temperature is not critical to the invention. In general, it has been found convenient to carry out the reaction at a temperature from about room temperature (20°C) to 150°C, preferably of from 20°C to the boiling temperature of the solvent, more preferably 90°C.

The reaction can be conducted for a time sufficient to obtain a satisfactory reaction rate, usually between 1 and 10 hours.

### Step iii)

The compounds thus prepared may be recovered from the reaction mixture by conventional means, for example the compounds may be recovered by distilling of the solvent from the reaction mixture or, if necessary, optionally after distilling of the solvent from the reaction mixture, pouring the residue into water, followed by extraction with a water-immiscible organic solvent and distilling of the solvent from the extract. Additionally, the product can, if desired, be further purified by various well known techniques, such as recrystallization, reprecipitation or the various chromatography techniques, notably column chromatography or preparative thin layer chromatography.

The following scheme 4 is given as an illustration of the method for preparing the compounds of formula (I) but is not to be considered as limiting the invention in its scope.

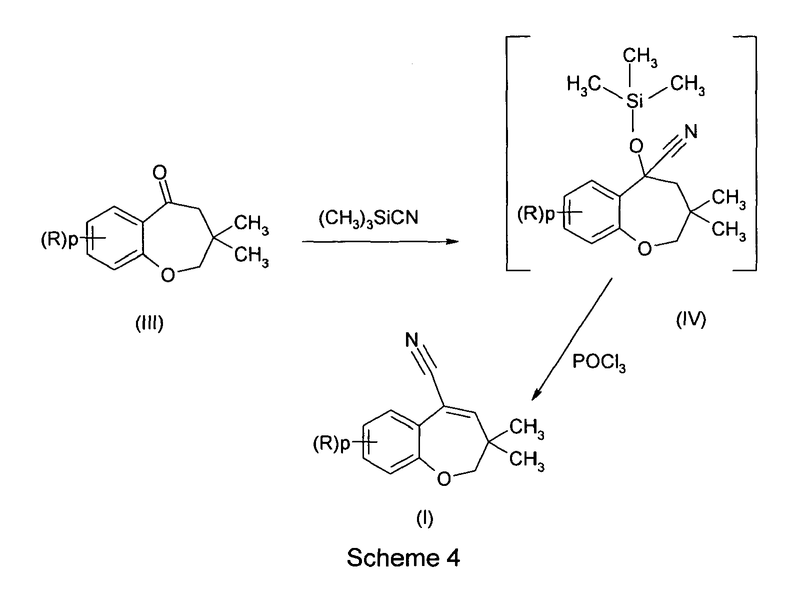

The invention is illustrated by the following representative and non-limiting examples :

### Example 1 : 3,3-dimethyl-5-cyano-7-methoxy-2,3-dihydro-benzoxepine (IA) :

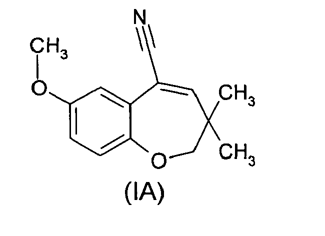

A mixture of compound (III) (1 kg, 4.54 moles), trimethylsilylcyanide (0.518 kg, 1.15 eq.) and n butyllithium 2.5 M in hexane (0.031 kg, 0.025 eq.) in pyridine is stirred at 20-25°C for 5 hours. Then, phosphorus oxychloride (1.044 kg, 1.5 eq.) is added and the mixture is heated at approximately 90°C for 6 hours.

The reaction mixture is cooled to 50-60°C and poured onto a mixture of toluene and water previously cooled to approximately 0°C. After stirring at 20-25°C for half an hour, the aqueous phase is separated and the organic phase is washed successively with diluted aqueous sodium hydroxide - sodium hypochlorite mixture, aqueous sodium chloride, diluted aqueous sulphuric acid and aqueous sodium chloride. The toluene solution is finely partially concentrated at atmospheric pressure, treated with active charcoal and used without further purification (yield : 65-83 %).
MP : 72°C.
¹HRMNδ ppm : 1.08(6H,s); 3.7(3H,s); 3.8(2H,s); 6.8-7.1(4H,m)
¹³CRMNδppm : 24.9; 42.6; 55.9; 78.5; 109.9; 115.9; 119.3; 122; 122.4; 153.4; 155.1; 157.3

### Example 2 : 3,3-dimethyl-5-formyl-7-methoxy-2,3-dihydrobenzoxepine (formula (II) : R = methoxy, p=1)

A toluene solution of compound (IA) (1 kg, 4.36 moles) is cooled to approximately -10°C and a 20% toluene solution of diisobutylaluminum hydride (3.41 kg, 1.1 eq.) is added while maintaining the temperature between -10°C and 0°C. The mixture is stirred at this temperature for 1 hour and the end of the reaction is controlled by TLC.

The reaction mixture is then poured onto 5N hydrochloric acid at such a rate that the temperature does not exceed 40°C. The mixture is stirred for 1 hour and the aqueous phase is separated. The organic phase is washed with water and concentrated to dryness under vacuum. The residue is dissolved in ethanol and treated with an aqueous solution of sodium metabisulfite (1.32 kg, 1.6 eq.) at reflux for 3 hours. The end of the reaction is controlled by TLC and the ethanol is removed by distillation. The solution obtained is cooled, washed with toluene at approximately 30°C, cooled to 15°C and basified with 30% aqueous sodium hydroxide. The suspension is stirred at approximately 15°C for half an hour and the solid is separated, washed with water and dried at 50-55°C (yield : 68-78%). This compound was identical to the compound obtained in example 16i) of EP 1140893 B1.

## Claims

1. Compounds of general formula (I) : 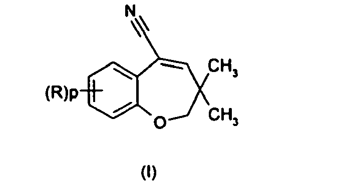 in which
each of R is independently chosen from a halogen atom; a cyano group; a nitro group; a carboxy group; an optionally halogenated (C₁-C₁₈)alkoxycarbonyl group; an Rₐ-CO-NH- or RₐR_{b}N-CO- group [in which Rₐ and R_{b} independently represent optionally halogenated (C₁-C₁₈)alkyl; a hydrogen atom; (C₆-C₁₀)aryl or (C₆-C₁₀)aryl(C₁-C₅)alkyl (where the aryl parts are optionally substituted by a halogen atom, by an optionally halogenated (C₁-C₅)alkyl group or by an optionally halogenated (C₁-C₅)alkoxy group); (C₃-C₁₂)cycloalkyl optionally substituted by a halogen atom, by an optionally halogenated (C₁-C₅)alkyl group or by an optionally halogenated (C₁-C₅)alkoxy group]; an optionally halogenated (C₁-C₁₈)alkyl group; optionally halogenated (C₁-C₁₈)alkoxy; and (C₆-C₁₀)aryl, (C₆-C₁₀)aryi(C₁-C₅)alkyl, (C₆-C₁₀)aryloxy, (C₃-C₁₂)cyclo-alkyl, (C₃-C₁₂)cycloalkenyl, (C₃-C₁₂)cycloalkyloxy, (C₃-C₁₂)cycloalkenyloxy; (C₆-C₁₀)aryloxycarbonyl or (C₆-C₁₀)arylcarbonyl;
in which the aryl, cycloalkyl and cycloalkenyl parts are optionally substituted by a halogen atom, by an optionally halogenated (C₁-C₅)alkyl or by an optionally halogenated (C₁-C₅)alkoxy;
R may be located in position 6, 7, 8 or 9 on the benzoxepine structure;
p represents 0, 1, 2, 3 or 4;
as well as their geometric isomers and stereoisomers of the compounds of formula (I) or mixtures thereof.

2. Compounds of formula (I) according to claim 1, wherein each of R independently represents a halogen atom, an optionally substituted halogenated (C₆-C₁₀) arylcarbonyl, an optionally halogenated (C₁-C₁₈) alkyl, an optionally halogenated (C₁-C₁₈) alkoxy, or an optionally halogenated (C₆-C₁₀) aryl.

3. Compounds according to claim 1 or 2, wherein R represents a (C₁-C₄) alkoxy group.

4. Compounds according to anyone of the preceding claims, wherein R represents a methoxy group.

5. Compounds according to anyone of the preceding claims, wherein p is 1 or 2.

6. Compounds according to anyone of the preceding claims, wherein p is 1.

7. Compounds according to anyone of the preceding claims chosen from:
3,3-dimethyl-5-cyano-7-bromo-2,3-dihydrobenzoxepine,
3,3-dimethyl-5-cyano-9-methoxy-2,3-dihydrobenzoxepine,
3,3-dimethyl-5-cyano-7,8-dichloro-2,3-dihydrobenzoxepine,
3,3-dimethyl-5-cyano-7-fluoro-8-chloro-2,3-di-hydrobenzoxepine,
3,3-dimethyl-5-cyano-7-(para-chlorobenzoyl)-2,3-dihydrobenzoxepine,
3,3-dimethyl-5-cyano-7-trifluoromethyl-2,3-di-hydrobenzoxepine,
3,3-dimethyl-5-cyano-7-fluoro-2,3-dihydrobenzoxepine,
3,3-dimethyl-5-cyano-7-chloro-2,3-dihydrobenzoxepine,
3,3-dimethyl-5-cyano-7,8-dimethoxy-2,3-dihydro-benzoxepine,
3,3-dimethyl-5-cyano-7-phenyl-2,3-dihydrobenzoxepine,
3,3-dimethyl-5-cyano-2,3-dihydrobenzoxepine,
3,3-dimethyl-5-cyano-7-methoxy-2,3-dihydrobenzoxepine
as well as their geometric isomers and stereoisomers or mixtures thereof.

8. Compound according to anyone of the preceding claims which is 3,3-dimethyl-5-cyano-7-methoxy-2,3-dihydrobenzoxepine, as well as its geometric isomers and stereoisomers or mixtures thereof.

9. Process of preparation of compounds of formula (I) according to anyone of the preceding claims comprising :
i) reacting a compound of formula (III) : 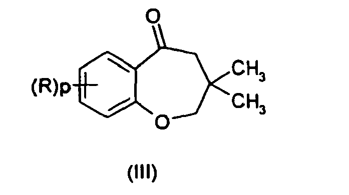 with CN⁻ or a species providing CN-;
ii) hydrolyzing the resulting mixture.

10. Process according to claim 9, wherein in step i), the compound of formula (III) is reacted with an alkali metal cyanide, diethylaluminum cyanide (Et₂AlCN) or trialkyl- or triaryl-silylcyanide.

11. Process according to claim 9 or 10, wherein in step i), the compound of formula (III) is reacted with a trialkylsilylcyanide.

12. Process according to claim 11, wherein said trialkylsilylcyanide is Me₃SiCN.

13. Process according to claim 11 or 12, wherein the reaction of step i) is conducted in the presence of a Lewis acid or a base.

14. Process according to claim 13, wherein said base is chosen from alkali metal hydrides; (C₁-C₁₀) alkyllithium compounds; alkali metal alkoxides; and alkali metal carbonates.

15. Process according to claim 13 or 14, wherein said base is chosen from alkyllithium.

16. Process according to anyone of claims 11 to 15, wherein step ii) is performed in the presence of a chlorinating agent.

17. Process according to claim 16, wherein said chlorinating agent is chosen from phosphorus oxychloride (POCl₃), thionyl chloride (SOCl₂), sulfonide chloride (SO₂Cl₂).

18. Process according to anyone of claims 9 to 17 which further comprises the step:
iii) isolating the obtained compound of formula (I).

19. Process of preparation of a compound of formula (II): 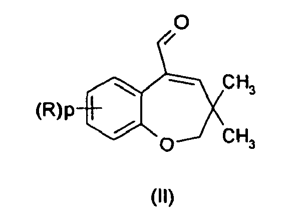 in which R and p are defined as in anyone of claims 1 to 8, said process comprising :
a) reacting a compound of formula (I) as defined in anyone of claims 1 to 8 with a reducing agent.

20. Process according to claim 19, wherein step a) is carried out in the presence of a reducing agent.

21. Process according to claim 20, wherein said reducing agent is chosen from LiAIH₄, LiAIH(Oalkyl)₃, LiAIH₂(Oalkyl)₂, LiAIH(NR₂)₃ where R is H or alkyl, and DIBAL-H.

22. Process according to claim 20 or 21, wherein said reducing agent is DIBAL-H.

23. Process according to anyone of claims 19 to 22 which further comprises the step :
b) hydrolyzing the resulting mixture.

24. Process according to claim 23, wherein step b) is performed under acid conditions.

25. Process according to anyone of claims 19 to 24 which further comprises the step:
c) isolating the obtained compound of formula (II).

## Patentansprüche

1. Verbindungen mit der allgemeinen Formel (I): 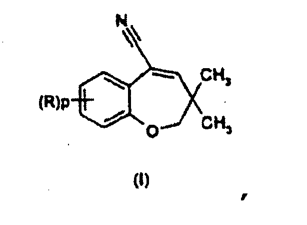 in welcher jeder R unabhängig voneinander aus einem Halogenatom, einer Cyanogruppe, einer Nitrogruppe, einer Carboxygruppe, einer wahlweise halogenierten (C₁- bis C₁₈-) Alkoxycarbonylgruppe, einer Rₐ-CO-NH- oder RₐR_{b}N-CO-Gruppe [in welcher Rₐ und R_{b} unabhängig voneinander wahlweise halogeniertes (C₁- bis C₁₈-)Alkyl, ein Wasserstoffatom, (C₆- bis C₁₀-)Aryl oder (C₆- bis C₁₀-)Aryl(C₁- bis C₅-)Alkyl (wobei die Arylteile wahlweise mit einem Halogenatom, einer wahlweise halogenierten (C₁- bis C₅-)Alkylgruppe oder einer wahlweise halogenierten (C₁- bis C₅-)Alkoxygruppe substituiert sind) und (C₃- bis C₁₂-)Cycloalkyl, das wahlweise mit einem Halogenatom, einer wahlweise halogenierten (C₁- bis C₅-)Alkylgruppe oder einer wahlweise halogenierten (C₁- bis C ₅- )Alkoxygruppe substituiert ist, bedeuten], einer wahlweise halogenierten (C₁- bis C₁₈-) Alkylgruppe, einem wahlweise halogenierten (C₁- bis C₁₈-) Alkoxy und ( C₆- bis C1o-)Aryl, ( C₆- bis C₁₀-)Aryl(C₁- bis C₅- ) Alkyl , ( C₆- bis C₁₀-) Aryloxy, (C₃- bis C₁₂-)Cycloalkyl, (C₃- bis C₁₂-)Cycloalkenyl, (C₃- bis C₁₂-)Cycloalkyloxy, (C₃- bis C₁₂-)Cycloalkenyloxy, (C₆- bis C₁₀-)Aryloxycarbonyl oder (C₆- bis C₁₀-)Arylcarbonyl ausgewählt ist, wobei die Aryl-, Cycloalkyl- und Cycloalkenylteile wahlweise mit einem Halogenatom, einem wahlweise halogenierten (C₁- bis C₅-) Alkyl oder einem wahlweise halogenierten (C₁- bis C₅-)Alkoxy substituiert sind,
R sich in 6-, 7-, 8- oder 9-Position an der Benzoxepinstruktur befinden kann und
p 0, 1, 2, 3 oder 4 bedeutet,
sowie die geometrischen Isomeren und Stereoisomeren der Verbindungen mit der Formel (I) oder Gemische davon.

2. Verbindungen mit der Formel (I) nach Anspruch 1, wobei jeder R unabhängig voneinander ein Halogenatom, ein wahlweise substituiertes halogeniertes (C₆- bis C₁₀-) Arylcarbonyl, ein wahlweise halogeniertes (C₁- bis C₁₈-)Alkyl, ein wahlweise halogeniertes (C₁- bis C₁₈-)Alkoxy oder ein wahlweise halogeniertes (C₆- bis C₁₀-) Aryl bedeutet.

3. Verbindungen nach Anspruch 1 oder 2, wobei R eine (C₁- bis C₄-) Alkoxygruppe bedeutet.

4. Verbindungen nach einem der vorhergehenden Ansprüche, wobei R eine Methoxygruppe bedeutet.

5. Verbindungen nach einem der vorhergehenden Ansprüche, wobei p 1 oder 2 bedeutet.

6. Verbindungen nach einem der vorhergehenden Ansprüche, wobei p 1 bedeutet.

7. Verbindungen nach einem der vorhergehenden Ansprüche, die aus:
3,3-Dimethyl-5-cyano-7-brom-2,3-dihydrobenzoxepin,
3,3-Dimethyl-5-cyano-9-methoxy-2,3-dihydrobenzoxepin,
3,3-Dimethyl-5-cyano-7,8-dichlor-2,3-dihydrobenzoxepin,
3,3-Dimethyl-5-cyano-7-fluor-8-chlor-2,3-dihydrobenzoxepin
3,3-Dimethyl-5-cyano-7-(p-chlorbenzoyl)-2,3-dihydrobenzoxepin,
3,3-Dimethyl-5-cyano-7-trifluormethyl-2,3-dihydrobenzoxepin,
3,3-Dimethyl-5-cyano-7-fluor-2,3-dihydrobenzoxepin,
3,3-Dimethyl-5-cyano-7-chlor-2,3-dihydrobenzoxepin,
3,3-Dimethyl-5-cyano-7,8-dimethoxy-2,3-dihydrobenzoxepin,
3,3-Dimethyl-5-cyano-7-phenyl-2,3-dihydrobenzoxepin,
3,3-Dimethyl-5-cyano-2,3-dihydrobenzoxepin und
3,3-Dimethyl-5-cyano-7-methoxy-2,3-dihydrobenzoxepin
sowie ihren geometrischen Isomeren und Stereoisomeren oder Gemischen davon ausgewählt sind.

8. Verbindung nach einem der vorhergehenden Ansprüche, die 3,3-Dimethyl-5-cyano-7-methoxy-2,3-dihydrobenzoxepin ist, sowie ihre geometrischen Isomeren und Stereoisomeren oder Gemische davon.

9. Verfahren zur Herstellung von Verbindungen mit der Formel (I) nach einem der vorhergehenden Ansprüche, welches das:
I) Umsetzen einer Verbindung mit der Formel (III): 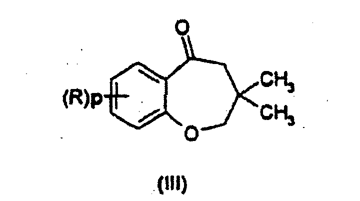 mit CN⁻ oder einer CN⁻ liefernden Spezies und
II) Hydrolysieren des erhaltenen Gemischs
umfasst.

10. Verfahren nach Anspruch 9, wobei in Stufe I) die Verbindung mit der Formel (III) mit einem Alkalimetallcyanid, Diethylaluminiumcyanid (Et₂AlCN) oder Trialkyl- bzw. Triarylsilylcyanid umgesetzt wird.

11. Verfahren nach Anspruch 9 oder 10, wobei in Stufe I) die Verbindung mit der Formel (III) mit einem Trialkylsilylcyanid umgesetzt wird.

12. Verfahren nach Anspruch 11, wobei das Trialkylsilylcyanid Me₃SiCN ist.

13. Verfahren nach Anspruch 11 oder 12, wobei die Umsetzung von Stufe I) in Gegenwart einer Lewis-Säure oder -Base durchgeführt wird.

14. Verfahren nach Anspruch 13, wobei die Base aus Alkalimetallhydriden, (C₁- bis C₁₀-)Alkyllithiumverbindungen, Alkalimetallalkoxiden und Alkalimetallcarbonaten ausgewählt ist.

15. Verfahren nach Anspruch 13 oder 14, wobei die Base aus Alkyllithium ausgewählt ist.

16. Verfahren nach einem der Ansprüche 11 bis 15, wobei die Stufe II) in Gegenwart eines Chlorierungsmittels durchgeführt wird.

17. Verfahren nach Anspruch 16, wobei das Chlorierungsmittel aus Phosphorchloridoxid (POCl₃), Thionylchlorid, (SOCl₂) und Sulfonidchlorid (SO₂Cl₂) ausgewählt ist.

18. Verfahren nach einem der Ansprüche 9 bis 17, das außerdem die Stufe:
III) Isolieren der erhaltenen Verbindung mit der Formel (I) umfasst.

19. Verfahren zur Herstellung einer Verbindung mit der Formel (II) : 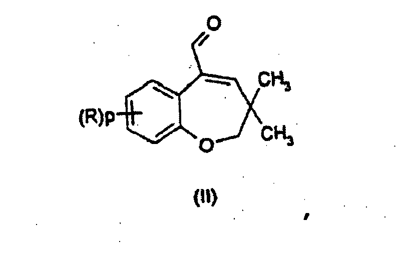 in welcher R und p wie in einem der Ansprüche 1 bis 8 definiert sind, wobei das Verfahren das
a) Umsetzen einer wie in einem der Ansprüche 1 bis 8 definierten Verbindung mit der Formel (I) mit einem Reduktionsmittel
umfasst.

20. Verfahren nach Anspruch 19, wobei die Stufe a) in Gegenwart eines Reduktionsmittels durchgeführt wird.

21. Verfahren nach Anspruch 20, wobei das Reduktionsmittel aus LiAlH₄ , LiAlH(Oalkyl)₃, LiAlH₂(Oalkyl)₂, LiAlH(NR₂)₃, wobei R H oder Alkyl bedeutet, und DIBAL-H ausgewählt ist.

22. Verfahren nach Anspruch 20 oder 21, wobei das Reduktionsmittel DIBAL-H ist.

23. Verfahren nach einem der Ansprüche 19 bis 22, das weiterhin die Stufe:
b) Hydrolysieren des erhaltenen Gemischs umfasst.

24. Verfahren nach Anspruch 23, wobei die Stufe b) unter sauren Bedingungen durchgeführt wird.

25. Verfahren nach einem der Ansprüche 19 bis 24, das ferner die Stufe:
c) Isolieren der erhaltenen Verbindung mit der Formel (II)
umfasst.

## Revendications

1. Composés de formule générale (I) : 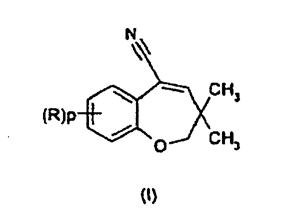 dans laquelle
chaque R est choisi indépendamment parmi un atome d'halogène ; un groupe cyano ; un groupe nitro ; un groupe carboxy ; un groupe (C₁-C₁₈)alcoxycarbonyle éventuellement halogéné ; un groupe Rₐ-CO-NH- ou RₐR_{b}N-CO- [dans lesquels Rₐ et R_{b} représentent indépendamment un (C₁-C₁₈)alkyle éventuellement halogéné ; un atome d'hydrogène; un (C₆-C₁₀)aryle ou un (C₆-C₁₀)aryle(C₁-C₅)alkyle (les parties aryles étant éventuellement substituées par un atome d'halogène, par un groupe (C₁-C₅)alkyle éventuellement halogéné ou par un groupe (C₁-C₅)alcolxy éventuellement halogéné) ; un (C₃-C₁₂)cycloalkyle éventuellement substitué par un atome d'halogène, par un groupe (C₁-C₅)alkyle éventuellement halogéné ou par un groupe (C₁-C₅)alcolxy éventuellement halogéné] ; un groupe (C₁-C₁₈)alkyle éventuellement halogéné ; un (C₁-C₁₈)alcoxy éventuellement halogéné ; et un (C₆-C₁₀)aryle, un (C₆-C₁₀)aryle(C₁-C₅)alkyle, un (C₆-C₁₀)aryloxy, un (C₃-C₁₂)cyclo-alkyle, un (C₃-C₁₂)cycloalcényle, un (C₃-C₁₂)cycloalkyloxy, un (C₃-C₁₂)cycloalcényloxy; un (C₆-C₁₀)aryloxycarbonyl ou un (C₆-C₁₀)arylcarbonyl;
dans laquelle les parties aryle, cycloalkyle et cycloalcényle sont éventuellement substituées par un atome d'halogène, par un (C₁-C₅)alkyle éventuellement halogéné ou par un (C₁-C₅)alcolxy éventuellement halogéné;
R peut être situé en position 6, 7, 8 ou 9 sur la structure benzoxépine ;
p représente 0, 1, 2, 3 ou 4 ;
ainsi que leurs isomères et stéréoisomères géométriques des composés de formule (I) ou leurs mélanges.

2. Composés de formule (I) selon la revendication 1, dans lesquels chaque R représente indépendamment un atome d'halogène, un (C₆-C₁₀)arylcarbonyle halogéné éventuellement substitué, un (C₁-C₁₈)alkyle éventuellement halogéné, un (C₁-C₁₈)alcoxy éventuellement halogéné ou un (C₆-C₁₀)aryle éventuellement halogéné.

3. Composés selon la revendication 1 ou 2, dans lesquels R représente un groupe (C₁-C₄)alcoxy.

4. Composés selon l'une quelconque des revendications précédentes, dans lesquels R représente un groupe méthoxy.

5. Composés selon l'une quelconque des revendications précédentes, dans lesquels p vaut 1 ou 2.

6. Composés selon l'une quelconque des revendications précédentes, dans lesquels p vaut 1.

7. Composés selon l'une quelconque des revendications précédentes, choisis parmi :
3,3-diméthyl-5-cyano-7-bromo-2,3-dihydrobenzoxépine,
3,3-diméthyl-5-cyano-9-méthoxy-2,3-dihydrobenzoxépine,
3,3-diméthyl-5-cyano-7,8-dichloro-2,3-dihydrobenzoxépine,
3,3-diméthyl-5-cyano-7-fluoro-8-chloro-2,3-di-hydrobenzoxépine,
3,3-diméthyl-5-cyano-7-(para-chlorobenzoyl)-2,3-dihydrobenzoxépine,
3,3-diméthyl-5-cyano-7-trifluorométhyl-2,3-dihydrobenzoxépine,
3,3-diméthyl-5-cyano-7-fluoro-2,3-dihydrobenzoxépine,
3,3-diméthyl-5-cyano-7-chloro-2,3-dihydrobenzoxépine,
3,3-diméthyl-5-cyano-7,8-diméthoxy-2,3-dihydrobenzoxépine,
3,3-diméthyl-5-cyano-7-phényl-2,3-dihydrobenzoxépine,
3,3-diméthyl-5-cyano-2,3-dihydrobenzoxépine,
3,3-diméthyl-5-cyano-7-méthoxy-2,3-dihydrobenzoxépine
ainsi que leurs isomères et stéréoisomères géométriques ou leurs mélanges.

8. Composé selon l'une quelconque des revendications précédentes, étant le 3,3-diméthyl-5-cyano-7-méthoxy-2,3-dihydrobenzoxépine, ainsi que ses isomères et stéréoisomères géométriques ou leurs mélanges.

9. Procédé de préparation des composés de formule (I) selon l'une quelconque des revendications précédentes, comprenant :
i) la mise en réaction d'un composé de formule (III) : 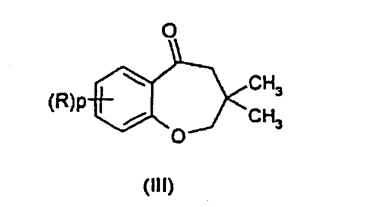 avec CN⁻ ou une espèce fournissant CN- ;
ii) l'hydrolyse du mélange obtenu.

10. Procédé selon la revendication 9, dans lequel lors de l'étape i), le composé de formule (III) est mis en réaction avec un cyanure de métal alcalin, un cyanure de diéthylaluminum (Et₂AlCN) ou un trialkyl- ou triaryl-silylcyanure.

11. Procédé selon la revendication 9 ou 10, dans lequel lors de l'étape i), le composé de formule (III) est mis en réaction avec un trialkylsilylcyanure.

12. Procédé selon la revendication 11, dans lequel ledit trialkylsilylcyanure est Me₃SiCN.

13. Procédé selon la revendication 11 ou 12, dans lequel la réaction de l'étape i) est réalisée en présence d'une base ou d'un acide de Lewis.

14. Procédé selon la revendication 13, dans lequel ladite base est choisie parmi les hydrures de métal alcalin ; les composés (C₁-C₁₀)alkyllithium ; les alcoxydes de métal alcalin ; et les carbonates de métal alcalin.

15. Procédé selon la revendication 13 ou 14, dans lequel ladite base est choisie parmi les alkyllithiums.

16. Procédé selon l'une quelconque des revendications 11 à 15, dans lequel l'étape ii) est réalisée en présence d'un agent chlorant.

17. Procédé selon la revendication 16, dans lequel ledit agent chlorant est choisi parmi l'oxychlorure de phosphore (POCl₃), le chlorure de thionyle (SOCl₂), le chlorure de sulfuryle (SO₂Cl₂).

18. Procédé selon l'une quelconque des revendications 9 à 17, comprenant en outre l'étape consistant à :
iii) isoler le composé de formule (I) obtenu.

19. Procédé de préparation d'un composé de formule (II) : 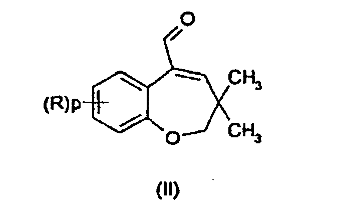 dans laquelle R et p sont tels que définis dans l'une quelconque des revendications 1 à 8, ledit procédé comprenant :
a) la mise en réaction d'un composé de formule (I) telle que définie dans l'une quelconque des revendications 1 à 8 avec un agent réducteur.

20. Procédé selon la revendication 19, dans lequel l'étape a) est réalisée en présence d'un agent réducteur.

21. Procédé selon la revendication 20, dans lequel ledit agent réducteur est choisi parmi LiAlH₄, LiAIH(Oalkyl)₃, LiAlH₂(Oalkyl)₂, LIAIH(NR₂)₃, R représentant H ou un alkyle, et DIBAL-H.

22. Procédé selon la revendication 20 ou 21, dans lequel ledit agent réducteur est DIBAL-H.

23. Procédé selon l'une quelconque des revendications 19 à 22, comprenant en outre l'étape consistant à :
b) hydrolyser le mélange obtenu.

24. Procédé selon la revendication 23, dans lequel l'étape b) est réalisée en conditions acides.

25. Procédé selon l'une quelconque des revendications 19 à 24, comprenant en outre l'étape consistant à :
c) isoler le composé de formule (II) obtenu.
